## Europäisches Patentamt

## European Patent Office

## Office européen des brevets

(11) Veröffentlichungsnummer: **0 355 177**

**A1**

# EUROPÄISCHE PATENTANMELDUNG

(21) Anmeldenummer: **88113375.5**

(22) Anmeldetag: **17.08.88**

(51) Int. Cl.⁴: **A61B 17/22 , G10K 11/30**

(43) Veröffentlichungstag der Anmeldung:
**28.02.90 Patentblatt 90/09**

(84) Benannte Vertragsstaaten:
**DE FR GB NL**

(71) Anmelder: **Siemens Aktiengesellschaft**
**Wittelsbacherplatz 2**
**D-8000 München 2(DE)**

(72) Erfinder: **Hassler, Dietrich, Dipl.-Ing.**
**Flugweg 3**
**D-8525 Uttenreuth(DE)**
Erfinder: **Schmidt, Erhard, Ing. grad.**
**Heuwaagstrasse 20**
**D-8520 Erlangen(DE)**

(54) **Einrichtung zum berührungslosen Zertrümmern von Konkrementen im Körper eines Lebewesens.**

(57) Die Erfindung betrifft eine Einrichtung zum berührungslosen Zertrümmern von Konkrementen (18) im Körper (4) eines Lebewesens mit einer Stoßwellenquelle (2) zur Erzeugung von in einer Fokuszone (F) zusammenlaufenden Stoßwellen, wobei die Stoßwellenquelle (2) wahlweise zur Erzeugung von Stoßwellen hoher Intensität zur Zertrümmerung eines Konkrementes (18) und zur Erzeugung von Stoßwellen geringer Intensität für bildgebende Zwecke antreibbar ist. Zwischen der Fokuszone (F) und der Stoßwellenquelle (2) ist ein mit einer Empfangsschaltung verbundener Drucksensor (24) angeordnet. Außerdem ist zwischen dem Drucksensor (24) und der Fokuszone eine akustische Ablenkeinrichtung angeordnet, mittels derer die Stoßwellen geringer Intensität zur Erzeugung eines die Fokuszone (F) der Stoßwellen hoher Intensität erfassenden Sektor-Scans ablenkbar sind. Die Empfangsschaltung ist zur Erzeugung von B-Bildern betreibbar.

FIG 1

EP 0 355 177 A1

## Einrichtung zum berührungslosen Zertrümmern von Konkrementen im Körper eines Lebewesens

Die Erfindung betrifft eine Einrichtung zum berührungslosen Zertrümmern von Konkrementen im Körper eines Lebewesens mit einer Stoßwellenquelle zur Erzeugung von Stoßwellen in einem akustischen Ausbreitungsmedium, welche in einer Fokuszone zusammenlaufen, wobei die Stoßwellenquelle wahlweise zur Erzeugung von Stoßwellen hoher Intensität zur Zertrümmerung eines Konkrementes und zur Erzeugung von Stoßwellen geringer Intensität für bildgebende Zwecke antreibbar ist, mit Mitteln zur akustischen Koppelung der Stoßwellenquelle mit dem Körper des Lebewesens, und mit einem Drucksensor, der die akustischen Echosignale der Stoßwellen empfängt und der zur Auswertung der Echosignale mit einer Empfangsschaltung verbunden ist.

Bei der Zertrümmerung von Konkrementen mittels derartiger Einrichtungen wird so vorgegangen, daß zunächst das zu zertrümmernde Konkrement, z.B. ein Nierenstein, mittels einer geeigneten Ortungsvorrichtung im Körper des Lebewesens lokalisiert wird. Die Einrichtung wird dann relativ zu dem Körper des Lebewesens so ausgerichtet, daß sich das zu zertrümmernde Konkrement in der Fokuszone befindet, worauf es mit Stoßwellen beaufschlagt wird. Diese üben mechanische Beanspruchungen auf das Konkrement aus, die bewirken, daß dieses zunächst in Fragmente und mit fortschreitender Behandlung in feinen Grieß zerfällt, der auf natürlichem Wege abgehen kann.

Eine Einrichtung der eingangs genannten Art ist aus der DE-OS 27 22 252 bekannt. Dort sind mehrere räumlich voneinander getrennte Drucksensoren vorgesehen, die die Ortung eines zu zertrümmernden Konkrementes gestatten. Mittels der Drucksensoren und der Empfangsschaltung ist es nämlich möglich, die nach Beaufschlagung des Konkrementes mit einer Stoßwelle geringer Intensität von diesem infolge von Beugungserscheinungen ausgehende Kugelwelle zu registrieren. Zuvor ist es jedoch erforderlich, anhand eines mittels eines außerdem vorhandenen Ultraschall-Sektor-Applikators erzeugten Ultraschallbildes die Einrichtung relativ zu dem Körper des Lebewesens so auszurichten, daß sich das Konkrement bereits im Bereich der Fokuszone der Stoßwellen befindet. Die bekannte Einrichtung gestattet es also, unter anderem mittels Stoßwellen geringer Intensität ein zu zertrümmerndes Konkrement zu orten. Es wäre jedoch wünschenswert, während der Behandlung Informationen über den bereits erzielten Zerstörungsgrad bzw. den Behandlungserfolg erhalten zu können, um erkennen zu können, in wieweit das zu zertrümmernde Konkrement bereits in Fragmente oder in Grieß zerfallen ist. Dies ist im Falle der bekannten Einrichtung nicht möglich, da die von dem Ultraschall-Sektor-Applikator ausgesandten Ultraschallwellen infolge ihrer geringen Wellenlänge nicht in eine Ansammlung von Fragmenten bzw. in Grieß eindringen können, sondern bereits an der dem Ultraschall-Sektor-Applikator zugewandten Grenzfläche der Ansammlung von Fragmenten bzw. des Grießes reflektiert werden. Auch anhand der mittels der Drucksensoren empfangenen Echosignale der Stoßwellen geringer Intensität ist eine Kontrolle des bereits erreichten Zertrümmerungsgrades nicht möglich, da die erhaltenen Informationen im wesentlichen denen gleichen, die mittels eines im A-Mode betriebenen Ultraschall-Applikators erhalten werden.

Der Erfindung liegt die Aufgabe zugrunde, eine Einrichtung der eingangs genannten Art so auszubilden, daß eine Kontrolle des Zertrümmerungsgrades bzw. des Behandlungserfolges möglich ist.

Nach der Erfindung wird diese Aufgabe dadurch gelöst, daß der Drucksensor in dem Ausbreitungsmedium zwischen der Fokuszone und der Stoßwellenquelle angeordnet ist und eine Sensorfläche aufweist, die die Querschnittsfläche der Stoßwellen im Bereich des Drucksensors abdeckt, daß zwischen dem Drucksensor und der Fokuszone eine akustische Ablenkeinrichtung in dem Ausbreitungsmedium angeordnet ist, mittels derer die Stoßwellen geringer Intensität zur Erzielung eines die Fokuszone der Stoßwellen hoher Intensität erfassenden Sektor-Scans ablenkbar sind, und daß die Empfangsschaltung zur Erzeugung von B-Bildern betreibbar ist. Die Erfindung beruht auf der Erkenntnis, daß Stoßwellen, also auch die zu bildgebenden Zwecken ausgesandten Stoßwellen geringer Intensität, wenigstens hinsichtlich ihrer Grundwelle wesentlich tieffrequenter als die zu Ortungszwecken gewöhnlich verwendeten Ultraschallwellen sind. Dies hat zur Folge, daß Stoßwellen leichter um kleine Hindernisse "kriechen", so daß grundsätzlich die Möglichkeit besteht, zum einen im Sinne der Ausbreitungsrichtung hintereinanderliegende Fragmente eines Konkrementes abzubilden und zum anderen in den bei der Zertrümmerung von Konkrementen entstehenden Grieß einzudringen. Da im Falle der Erfindung die Stoßwellen geringer Intensität mittels der akustischen Ablenkeinrichtung zur Erzielung eines die Fokuszone erfassenden Sektor-Scans ablenkbar sind, kann mittels des Drucksensors und der Empfangsschaltung ein einem Ultraschall-B-Bild vergleichbares Bild erzeugt werden, das eine Kontrolle des Zertrümmerungsgrades bzw. des Behandlungserfolges gestattet. Es wird zwar nur ein scharfes Bild des die Fokuszone unmittelbar umgebenden Bereiches erzeugt, dies reicht jedoch aus, um eine effektive Kontrolle des Zertrümmerungsgrades zu ermöglichen. Außerdem wird infolge des Umstandes, daß der Drucksensor die Querschnittsfläche der Stoßwellen im Bereich des Drucksensors abdeckt, bei der Bilderzeugung die gesamte Apertur der Stoßwellenquelle genutzt. Das zu zertrümmernde Konkrement und seine nähere Umgebung werden also sozusagen "mit den Augen der Stoßwellenquelle" betrachtet, so daß weitere Informationen anfallen, die mittels eines Ultraschall-Sektor-Applikators, der jeweils nur eine einzige Ebene abtastet, nicht erhalten werden können. So können z.B. im Ausbreitungsweg der Stoßwellen befindliche Hindernisse, z.B. Rippen oder im Bereich der Ankoppelstelle zwischen der Einrichtung und dem Körper des Lebewesens befindliche Störstellen (Luftblasen), erkannt werden. Diese Informationen werden jedoch unter Umständen nur durch die integrale Bildhelligkeit vermittelt, da eine scharfe Abbildung wie erwähnt nur im Tiefenbereich der Fokuszone der Stoßwellenquelle erfolgt. Falls die Ortung zu zertrümmernder Konkremente mit Hilfe eines Ultraschall-Sektor- Applikators erfolgt, besteht außerdem die Möglichkeit, die von dem Drucksensor stammenden elektrischen Signale in der Weise zu demodulieren, daß ein Videosignal entsteht, das in Helligkeitsmodulation längs der zugehörigen Strahlenrichtung in ein mittels des Ultraschall-Sektor-Applikators erzeugtes Ultraschall-B-Bild gebracht werden kann, so daß alle wesentlichen Informationen in einem einzigen Bild dargestellt sind. Es versteht sich, daß in der Regel durch geeignete Maßnahmen sichergestellt ist, daß die Stoßwellen hoher Intensität, die ebenfalls die akustische Ablenkeinrichtung durchlaufen, stets in einer ortsfesten Fokuszone zusammenlaufen. Im Gegensatz zu den Stoßwellen geringer Intensität werden also die Stoßwellen hoher Intensität nicht zur Erzielung eines Sektor-Scans abgelenkt. Vorzugsweise wird die Abgabe von Stoßwellen hoher Intensität so erfolgen, daß die Fokuszone auf der Winkelhalbierenden des durch den Sektor-Scan der Stoßwellen geringer Intensität erfaßten Sektors liegen. Grundsätzlich besteht aber auch die Möglichkeit, die Stoßwellen hoher Intensität mittels der akustischen Ablenkeinrichtung zu verlagern, z.B. um durch die Atemtätigkeit des zu behandelnden Lebewesens bedingten Verlagerungen eines zu zertrümmernden Konkrementes folgen zu können.

Eine besonders vorteilhafte Variante der Erfindung sieht vor, daß die Stoßwellenquelle derart antreibbar ist, daß sie zwischen jeweils zwei Stoßwellen hoher Intensität eine Vielzahl von Stoßwellen geringer Intensität bei hoher Folgefrequenz, z.B. 2 kHz, erzeugt. Auf diese Weise ist ein rascher Bildaufbau gewährleistet, der sich zwischen zwei Stoßwellen hoher Intensität vollzieht. Außerdem kann die Zertrümmerungswirkung jeder einzelnen Stoßwelle hoher Intensität kontrolliert werden, so daß die Behandlung sofort bei Erreichen des gewünschten Erfolges beendet werden kann, ohne daß das zu behandelnde Lebewesen unnötig mit weiteren Stoßwellen hoher Intensität belastet wird.

Gemäß einer bevorzugten Ausführungsform der Erfindung ist vor gesehen, daß die akustische Ablenkeinrichtung ein Paar von keilförmigen Körpern aufweist, die aus einem Werkstoff mit von der eines sie umgebenden akustischen Ausbreitungsmediums abweichender Schallausbreitungsgeschwindigkeit gebildet und in Ausbreitungsrichtung der Stoßwellen aufeinanderfolgend um Drehachsen relativ zueinander drehbar angeordnet sind, wobei jeder keilförmige Körper eine in bezug auf die Ausbreitungsrichtung der Stoßwellen um jeweils den gleichen Winkel geneigte und jeweils die Querschnittsfläche der Stoßwellen im Bereich der akustischen Ablenkeinrichtung abdeckende Begrenzungsfläche aufweist, und daß Mittel zum Drehen der keilförmigen Körper um die jeweilige Drehachse vorgesehen sind, mittels derer die keilförmigen Körper in entgegengesetzter Richtung mit gleicher Winkelgeschwindigkeit antreibbar sind, so daß während einer Umdrehung der keilförmigen Körper die Begrenzungsflächen in zwei Winkelpositionen parallel zueinander liegen. Eine derartige akustische Ablenkeinrichtung, die im Zusammenhang mit einer Ultraschall-Kamera in der US-PS 3 913 061 in allen Einzelheiten und hinsichtlich ihrer Wirkungsweise beschrieben ist, gestattet es auf einfache Weise, die Stoßwellen geringer Intensität zur Erzeugung des die Fokuszone der Stoßwellenquelle erfassenden Sektor-Scans abzulenken. Die keilförmigen Körper weisen übrigens vorzugsweise jeweils die gleichen Abmessungen auf und sind zweckmäßigerweise um eine gemeinsame Drehachse, vorzugsweise die Mittelachse der Stoßwellenquelle, drehbar.

Gemäß einer weiteren bevorzugten Ausführungsform der Erfindung sind als Drucksensor eine piezoelektrisch aktivierte Kunststoffolie, z.B. eine PVDF-Folie, und als Stoßwellenquelle eine stoßartig antreibbare Membran vorgesehen, wobei die Kunststoffolie auf der der Fokuszone zugewandten Seite der Membran, z.B. durch Kleben, aufgebracht ist. Es ergibt sich so ein einfacher konstruktiver Aufbau der Einrichtung. Außerdem werden dadurch durch Reflexionen der Echosignale in der Stoßwellenquelle verursachte negative Auswirkungen auf die Bilddynamik verringert, da sich das Mehrfachecho nicht darstellt. Vielmehr tritt lediglich eine Übertragungsnullstelle auf, die bei einer von der Dicke der Membran und der Schallausbreitungsgeschwindigkeit in dem Membranmaterial abhängigen Frequenz liegt. Die so entstehende Frequenzabhängigkeit stellt kein Problem dar, wenn die Übertragungsnullstelle auf eine geeignete Frequenz gelegt wird.

Gemäß einer Variante der Erfindung kann als Stoßwellenquelle eine piezoelektrische Stoßwellenquelle vorgesehen sein, die eine erste piezoelektrische Schicht zur Erzeugung der Stoßwellen und eine auf der ersten piezoelektrischen Schicht als Drucksensor aufgebrachte zweite piezoelektrische Schicht aufweist, die im Sinne der Ausbreitungsrichtung der Stoßwellen nach der ersten piezoelektrischen Schicht angeordnet ist und eine höhere Resonanzfrequenz aufweist als die erste piezoelektrische Schicht. Da die akustischen Echosignale infolge von Nicht-Linearitäten des Ausbreitungsmediums und des Körpers des Lebewesens hochfrequenter als die Grundschwingung der Stoßwellenquelle sind, ist durch diese Maßnahme eine ausreichende Empfindlichkeit des Drucksensors sichergestellt.

Gemäß einer Variante der Erfindung kann vorgesehen sein, daß mit dem Drucksensor empfangene Echosignale einer Stoßwelle hoher oder geringer Intensität mittels der Empfangsschaltung im A-Mode auswertbar sind. Da Stoßwellen im Vergleich zu Ultraschallwellen, wie sie von Ultraschall-Ortungseinrichtungen ausgehen, eine deutlich geringere Amplitudendämpfung erfahren, können auf diese Weise zusätzliche Informationen gewonnen werden. So können z.B. anhand der Höhe und Dauer des Echosignales insbesondere einer Stoßwelle hoher Intensität zusätzliche Informationen für die Ortung eines zu zertrümmernden Konkrementes und über die Größe des zu zertrümmernden Konkrementes erhalten werden. Außerdem können anhand der Echosignale insbesondere von Stoßwellen geringer Intensität zusätzliche Informationen über die Ankoppelstelle zwischen der Einrichtung und dem Körper des Lebewesens gewonnen werden. Bei Auswertung der Echosignale im A-Mode erfolgt die Aussendung einer Stoßwelle geringer Intensität vorzugsweise derart, daß diese in der gleichen Fokuszone wie die Stoßwellen hoher Intensität zusammenläuft, d.h., daß kein Sektor-Scan erfolgt.

Weitere Ausgestaltungen und Vorteile der Erfindung werden anhand der Beschreibung von in den beigefügten Figuren dargestellten Ausführungsbeispielen der Erfindung deutlich. Es zeigen:

Fig. 1 eine erfindungsgemäße Einrichtung im Längsschnitt,

Fig. 2 ein Blockschaltbild der Einrichtung nach Fig. 1, und

Fig. 3 eine weitere erfindungsgemäße Einrichtung im Längsschnitt.

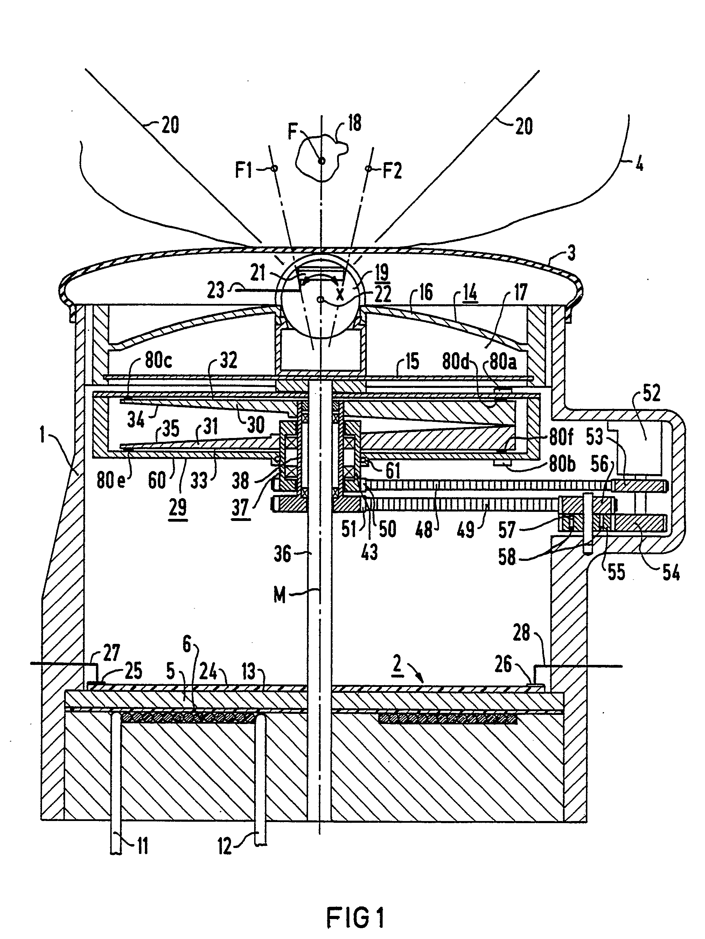

Die in Fig. 1 dargestellte erfindungsgemäße Einrichtung weist ein etwa rohrförmiges Gehäuse 1 auf, an dessen einem Ende eine insgesamt mit 2 bezeichnete Stoßwellenquelle vorgesehen ist. An seinem anderen Ende ist das Gehäuse 1 mittels eines flexiblen Balges 3 verschlossen, der dazu dient, die Einrichtung zur akustischen Koppelung an den im Querschnitt angedeuteten Körper 4 eines zu behandelnden Lebewesens anpressen zu können. Der Innenraum des Gehäuses 1 ist mit Wasser gefüllt, das als akustisches Ausbreitungsmedium vorgesehen ist.

Bei der Stoßwellenquelle 2 handelt es sich um eine elektro-dynamische Stoßwellenquelle, wie sie in der DE-OS 33 28 051 näher beschrieben ist. Die Stoßwellenquelle 2 weist eine ebene, kreisringförmige Membran 5 auf, deren eine Seite dem in dem Gehäuse befindlichen Wasser zugewandt ist. Der anderen Seite der aus einem elektrisch leitenden Werkstoff gebildeten Membran 5 gegenüberliegend ist eine Flächenspule 6 mit spiralförmig angeordneten Windungen vorgesehen, die über Anschlüsse 11, 12 mit einer noch zu beschreibenden Generatoreinrichtung mit Hochspannungsimpulsen beaufschlagbar ist. Wird die Flächenspule 6 mit einem Hochspannungsimpuls beaufschlagt, bewegt sich die Membran 5 von der Spule 6 schlagartig weg. Infolge dieser Bewegung wird in das Wasser ein im wesentlichen ebener Druckimpuls eingeleitet, der sich auf seinem Weg durch das Wasser zu einer Stoßwelle aufsteilt. Im folgenden wird der Einfachheit halber stets der Begriff Stoßwelle verwendet werden. Der zur Abstrahlung von Stoßwellen wirksame Bereich der Membran 5 wird im folgenden als Abstrahlfläche 13 der Membran 5 bezeichnet werden. Die Ausbreitungsrichtung der Stoßwellen entspricht der Richtung der Mittelachse M der Stoßwellenquelle 2.

Um die erzeugten ebenen Stoßwellen in der zur Zertrümmerung von Konkrementen erforderlichen Weise fokussieren zu können, ist im Wasser innerhalb des Gehäuses 1 eine insgesamt mit 14 bezeichnete akustische Sammellinse zwischen der Stoßwellenquelle 2 und dem Balg 3 angeordnet. Die akustische Sammellinse 14 ist als Flüssigkeitslinse ausgeführt. Sie besitzt also eine Eintrittswand 15 und eine Austrittswand 16, zwischen denen eine Linsenflüssigkeit 17 eingeschlossen ist, in der die Schallausbreitungsgeschwindigkeit von der Schallausbreitungsgeschwindigkeit in dem die Sammellinse 14 umgebenden Wasser abweicht. Wenn sich wie im Falle des dargestellten Ausführungsbeispieles in der Sammellinse 14 eine Linsenflüssigkeit 17 befindet, in der die Schallausbreitungsgeschwindigkeit geringer ist als in Wasser, muß die Sammellinse 14 plankonvex oder bikonvex ausgebildet sein. Im Falle des in Fig. 1 dargestellten Ausführungsbeispieles ist eine plankonvexe Sammellinse 14 vorgesehen. Durchläuft eine ebene Stoßwelle, deren Wellenfront im wesentlichen parallel zur Eintrittswand 15 verläuft, die akustische Sammellinse 14, wird die Stoßwelle auf eine mit F bezeichnete Fokuszone fokussiert, die auf der Mittelachse M der Stoßwellenquelle 2 liegt. Die Eintrittswand der Sammellinse 14 besteht übrigens aus Polymethylpentene (TPX) während die Ausgangswand aus Teflon (eingetragenes Warenzeichen) besteht. Als Linsenflüssigkeit ist eine Fluor-Kohlenstoff-Flüssigkeit, z.B. Flutec PP3 oder Fluorinert FC 75 (eingetragene Warenzeichen), vorgesehen.

Um die Einrichtung und den Körper 4 des zu behandelnden Lebewesens relativ zueinander so ausrichten zu können, daß ein im Körper 4 des zu behandelnden Lebewesens befindliches Konkrement 18 sich wie in der Fig. 1 dargestellt im Fokus F der Stoßwellen befindet, ist ein an sich bekannter und somit nur schematisch angedeuteter Ultraschall-Sektor-Applikator 19 vorhanden, der es im Zusammenwirken mit einer an sich bekannten und in Fig. 1 nicht dargestellten elektronischen Einrichtung gestattet, Ultraschall-B-Bilder von einer die Mittelachse M der Stoßwellenquelle 2 und somit die Fokuszone F enthaltenden, durch Linien 20 angedeuteten sektorförmigen Schicht des Körpers 4 des Lebewesens zu erstellen. Anhand dieses Bildes werden die Einrichtung und der Körper 4 des behandelnden Lebewesens so zueinander ausgerichtet, daß sich das zu zertrümmernde Konkrement 18 in der Fokuszone F der Stoßwellen befindet. Die mechanischen Bauteile des Ultraschall-Sektor-Applikators 19, von denen nur ein Ultraschall-Transducer 21 schematisch angedeutet ist, der um eine Achse 22 eine oszillierende Schwenkbewegung in Richtung des Doppelpfeiles X ausführt, wie sie zur Abtastung des durch die Linien 20 begrenzten Sektors erforderlich ist, sind in einer flüssigkeitsgefüllten Kapsel im Zentrum der akustischen Sammellinse 14 angeordnet. Der Ultraschall-Sektor-Applikator 19 steht über elektrische Leitungen, von denen der Übersichtlichkeit halber nur eine dargestellt und mit 23 bezeichnet ist, mit der erwähnten elektronischen Einrichtung in Verbindung.

Auf die dem in dem Gehäuse 1 befindlichen Wasser zugewandte Seite der Membran 5 ist durch Kleben eine Kunststoffolie 24 aufgebracht, die in ihrem die Abstrahlfläche 13 der Membran 5 abdeckenden Bereich piezoelektrisch aktiviert ist. Es handelt sich hierbei vorzugsweise um eine PVDF-Folie, deren Dicke ebenso wie die Dicke der Membran 5 übertrieben dargestellt ist. Zur elektrischen Kontaktierung ihres piezoelektrischen aktivierten Bereiches ist die Kunststoffolie 24 mit Metallisierungen 25, 26 versehen, an denen elektrische Leitungen 27, 28 angebracht sind, die zu einer noch zu beschreibenden Empfangsschaltung führen. Die Kunststoffolie 24 dient als Drucksensor, der zusammen mit der erwähnten Empfangsschaltung die Auswertung der akustischen Echosignale von in den Körper 4 des zu behandelnden Lebewesens eingeleiteten Stoßwellen gestattet. Der piezoelektrisch aktivierte Bereich der Kunststoffolie 24 dient als Sensorfläche und deckt, da er sich über die gesamte Abstrahlfläche 13 der Membran 5 erstreckt, die Querschnittsfläche der Stoßwellen im Bereich der Kunststoffolie 24 ab. Da die Kunststoffolie 24 auf der Membran befestigt ist, ist deren piezoelektrisch aktivierter Bereich in einer parallel zur Abstrahlfläche 13 verlaufenden Fläche angeordnet.

Zwischen der Kunststoffolie 24 und der Fokuszone F bzw. der akustischen Sammellinse 17 ist eine insgesamt mit 29 bezeichnete akustische Ablenkeinrichtung, deren Aufbau und Wirkungsweise noch beschrieben werden wird, in dem in dem Gehäuse 1 befindlichen Wasser angeordnet. Wesentlich ist zunächst nur, daß es die Ablenkeinrichtung 29 gestattet, die Stoßwellen derart abzulenken, daß die Fokuszone F der Stoßwellen längs einer etwa kreisbogenförmig gekrümmten, in einer die Mittelachse M der Stoßwellenquelle 2 enthaltenden Ebene liegenden Kurve zwischen den Positionen F1 und F2 in einer periodischen oszillierenden Bewegung verschoben wird. Es ist somit möglich, von der Abstrahlfläche 13 der Stoßwellenquelle 2 ausgehende Stoßwellen zur Erzielung eines Sektor-Scans abzulenken. Die akustischen Echosignale der abgelenkten Stoßwellen können dann mittels der als Drucksensor dienenden Kunststoffolie und der dieser zugeordneten Empfangsschaltung für bildgebende Zwecke ausgewertet werden. Vorzugsweise werden mittels der akustischen Ablenkeinrichtung 29 solche Stoßwellen zur Erzielung eines Sektor-Scans abgelenkt, die eine geringere Intensität als diejenigen Stoßwellen aufweisen, die zur Zertrümmerung eines Konkrementes erzeugt werden.

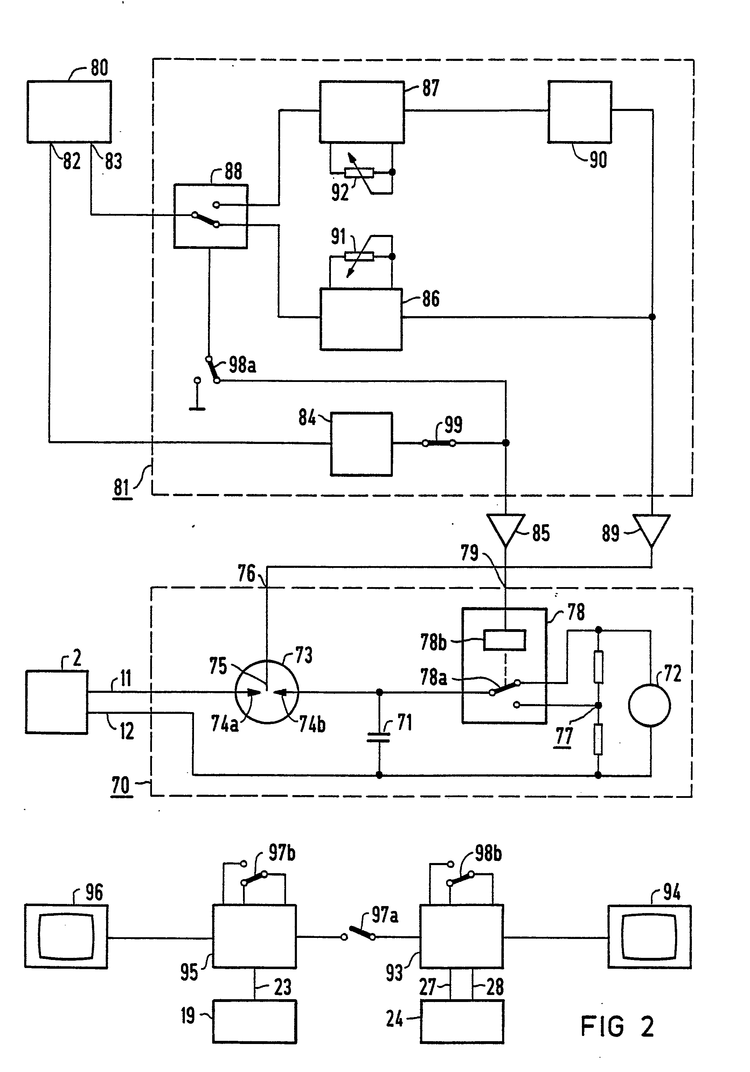

Gemäß Fig. 2 ist eine insgesamt mit 70 bezeichnete Generatoreinrichtung zum Antrieb der in Fig. 2 schematisch angedeuteten Stoßwellenquelle 2 vorgesehen, die es gestattet, Stoßwellen geringer und Stoßwellen hoher Intensität zu erzeugen. Wie aus der Fig. 2 ersichtlich ist, enthält die Generatoreinrichtung 70 einen Kondensator 71, der mittels einer Hochspannungsquelle 72 aufladbar ist. Zur Erzeugung einer Stoßwelle wird der Kondensator 71 mittels einer triggerbaren Funkenstrecke 73 an die Stoßwellenquelle 2 angeschaltet, in die er sich impulsartig entlädt. Zu diesem Zweck weist die Funkenstrecke 73 außer den Hauptelektroden 74a, 74b eine Hilfselektrode 75 auf, die mit einem Triggereingang 76 der Generatoreinrichtung verbunden ist. Wird der Hilfselektrode 75 ein Triggerimpuls zugeführt, bewirkt dieser eine Ionisierung der zwischen den Hauptelektroden 74a, 74b befindlichen Gasmoleküle, was zur Zündung der Funkenstrecke 73 führt. Parallel zu der Hochspannungsquelle 72 ist ein aus zwei Hochspannungswiderständen gebildeter Spannungsteiler 77 geschaltet. Mittels des Kontaktes 78a eines Hochspannungsrelais 78 besteht die Möglichkeit, den Kondensator 71 entweder direkt mit der Hochspannungsquelle 72 oder mit dem Abgriff des Spannungsteilers 77 zu verbinden. Je nach Schaltstellung des Hochspannungsrelais 78 besteht also die Möglichkeit, den Kondensator 71 entweder zur Erzeugung einer Stoßwelle hoher Intensität auf die volle von der Hochspannungsquelle 72 abgegebene Spannung oder zur Erzeugung einer Stoßwelle geringer Intensität auf eine entsprechend dem Spannungsteiler-Verhältnis verringerte Spannung aufzuladen. Die Erregerspule 78b des Hochspannungsrelais 78 ist mit einem Steuereingang 79 der Generatoreinrichtung verbunden.

Um die Einrichtung in der Weise betreiben zu können, daß die Stoßwellen hoher Intensität jeweils in einer ortsfesten Fokuszone zusammenlaufen und die Stoßwellen geringer Intensität in der zur Erzielung eines Sektor-Scans erforderlichen Weise abgelenkt werden, sind eine noch näher zu beschreibende, mit der akustischen Ablenkeinrichtung 29 gekoppelte Gebereinrichtung 80 und eine Steuereinrichtung 81 vorhanden, wobei die Steuereinrichtung 81 anhand von periodischen Signalen der Gebereinrichtung 80 die für den Triggereingang 76 und den Steuereingang 79 der Generatoreinrichtung 70 benötigen Signale erzeugt.

Die Gebereinrichtung 80 besitzt einen ersten Ausgang 82, an dem für jede Verschiebung der Fokuszone zwischen den Positionen F1 und F2 und umgekehrt ein Impuls auftritt. Außerdem ist ein zweiter Ausgang 83 vorhanden, an dem jedesmal dann, wenn die Fokuszone eine bestimmte Position einnimmt, ein Impuls auftritt. Die an den Ausgängen 82 und 83 zur Verfügung stehenden Signale sind der Steuereinrichtung 81 zugeführt.

Die Steuereinrichtung 81 weist eine digitale Frequenzteiler-Schaltung 84 auf, deren Eingang mit dem Ausgang 82 der Gebereinrichtung 80 in Verbindung steht. Im einfachsten Fall arbeitet die Frequenzteiler-Schaltung 84 mit dem Teilerfaktor "2", so daß an ihrem Ausgang ein Signal zur Verfügung steht, das seinen logischen Zustand mit jeder Verschiebung der Fokuszone von F1 nach F2 und umgekehrt ändert. Dieses Signal wird über einen Verstärker 85 der Erregerwicklung 78b des Hochspannungsrelais 78 zugeführt. Besitzt das Ausgangsignal der Frequenzteiler-Schaltung 84 den Wert logisch "0", nimmt der Kontakt 78a des Hochspannungsrelais 78 die in Fig. 2 dargestellte Schaltposition ein, d.h. der Kondensator 71 wird, wie es zur Erzeugung einer Stoßwelle hoher Intensität erforderlich ist, auf die volle von der Hochspannungsquelle 72 gelieferte Hochspannung aufgeladen. Besitzt das Ausgangsignal der Frequenzteiler-Schaltung 84 den Wert logisch "1", wechselt der Kontakt 78a des Hochspannungsrelais 78 seine Schaltposition, d.h. der Kondensator 71 ist mit dem Abgriff des Spannungsteilers 77 verbunden, wie es zur Erzeugung von Stoßwellen geringer Intensität erforderlich ist.

Außerdem enthält die Steuereinrichtung 81 zwei Impulsverzögerungsschaltungen 86, 87, deren Eingänge wechselweise mittels eines elektronischen Umschalters 88 mit dem Ausgang 83 der Gebereinrichtung 80 verbindbar sind. Der Umschalter 88 wird mittels des Ausgangssignales der Frequenzteiler-Schaltung 84 betätigt. Besitzt das Ausgangssignal der Frequenzteiler-Schaltung 84 des Wert logisch "0", nimmt der elektronische Umschalter 88 die in Fig. 2 dargestellte Schaltposition ein, d.h. die Impulsverzögerungsschaltung 86 ist mit dem Ausgang 83 der Gebereinrichtung 80 verbunden. Nimmt das Ausgangssignal der Frequenzteiler-Schaltung 84 den Wert logisch "1" an, wechselt der elektronische Umschalter 88 seine Schaltposition und die Impulsverzögerungsschaltung 87 ist mit dem Ausgang 83 der Gebereinrichtung 80 verbunden. Die Impulsverzögerungsschaltungen 86, 87 verzögern einen von dem Ausgang 83 der Gebereinrichtung 80 stammenden Impuls jeweils um eine bestimmte Impulsverzögerungszeit, die für die Impulsverzögerungsschaltungen 86, 87 unterschiedlich ist. Vom Ausgang der Impulsverzögerungsschaltung 86 gelangt der verzögerte Impuls über einen Verstärker 89 zu dem Triggereingang 76 der Generatoreinrichtung 70 und führt zur Zündung der Funkenstrecke 73 und damit zur Abgabe einer Stoßwelle. Da der elektronische Umschalter 88 ebenso wie das Hochspannungsrelais 78 durch das Ausgangssignal der Frequenzteiler-Schaltung 84 betätigt wird, löst ein von der Impulsverzögerungsschaltung 86 stammender Impuls eine Stoßwelle hoher Intensität aus. Die Impulsverzögerungsschaltung 87 dient zur Auslösung von Stoßwellen geringer Intensität. Zwischen die Impulsverzögerungsschaltung 87 und den Verstärker 89 ist ein Impulsgenerator 90 geschaltet, der derart ausgebildet ist, daß er dann, wenn seinem Eingang ein von der Impulsverzögerungschaltung 87 stammender Impuls zugeführt wird, an seinem Ausgang eine rasche Folge von beispielsweise 250 Impulsen erscheint. Die Folge von Impulsen erstreckt sich über eine Zeitdauer, die wesentlich geringer ist als die Periodendauer des am Ausgang 82 der Gebereinrichtung 80 auftretenden Signals. Während also jeweils nur einzelne Stoßwellen hoher Intensität erzeugt werden, wird zwischen zwei aufeinanderfolgenden Stoßwellen hoher Intensität eine der Anzahl der Impulse entsprechenden von Stoßwellen geringer Intensität erzeugt. Diese werden infolge der periodischen Ablenkung, die die akustische Achse der Stoßwellenquelle hinter der Ablenkeingrichtung 29 erfährt, nach Art eines Sektor-Scans abgelenkt. Dabei ist die Verzögerungszeit der Impulsverzögerungsschaltung 87 derart gewählt, daß die Abgabe der Stoßwellen geringer Intensität jeweils während einer Bewegung der Fokuszone zwischen den Positionen F1 und F2 oder umgekehrt erfolgt, und zwar vorzugsweise derart, daß ein Sektor abgetastet wird, dessen Winkelhalbierende der Mittelachse M der Stoßwellenquelle 2 entspricht. Die Verzögerungszeit der Impulsverzögerungsschaltung 86 ist derart gewählt, daß die Stoßwellen hoher Intensität jeweils in einer ortsfesten Fokuszone zusammenlaufen, die vorzugsweise auf der Mittelachse der Einrichtung liegt. Wie durch mit den Impulsverzögerungsschaltungen 86, 87 verbundene Einstellwiderstände 91, 92 angedeutet ist, besteht die Möglichkeit, die jeweiligen Impulsverzögerungszeiten einzustellen, um sie an die jeweiligen Bedürfnisse anpassen zu können.

Die akustischen Echosignale der Stoßwellen geringer Intensität werden mittels der Kunststoffolie 24, die in Fig. 1 schematisch angedeutet ist, aufgenommen und mittels der mit dieser verbundenen Empfangsschaltung 93 derart ausgewertet, daß ein einem Ultraschall-B-Bild ähnelndes Bild erhalten wird, das auf einem mit der Empfangsschaltung 93 verbundenen Datensichtgerät 94 dargestellt wird.

In der Fig. 2 ist außerdem der Ultraschall-Sektor-Applikator 19 schematisch angedeutet, der mit einer elektronischen Einrichtung 95 zur Erzeugung von Ultraschall-B-Bildern verbunden ist, die auf einem Datensichtgerät 96 dargestellt werden. Werden die in nicht dargestellter Weise miteinander gekoppelten Umschalter 97a, 97b ausgehend von der in Fig. 1 dargestellten Schaltposition in die jeweils andere Schaltposition gebracht, werden die mittels der Empfangsschaltung 93 erzeugten Signale über den Umschalter 97a der elektronischen Einrichtung 95 zugeführt, die gleichzeitig durch den Umschalter 97b, in eine solche Betriebsweise umgeschaltet wird, daß die mittels der Kunststoffolie 24 und der Empfangsschaltung 93 gewonnenen Informationen in Helligkeitsmodulation längs der zugehörigen Strahlenrichtung in das mittels des Ultraschall-Sektor-Applikators 19 und der elektronischen Einrichtung 95 erzeugte Ultraschall-B-Bild gebracht werden.

Eine von der zuvor beschriebenen abweichende Betriebsweise er gibt sich, wenn die in nicht dargestellter Weise miteinander gekoppelten Umschalter 98a, 98b ausgehend von der in Fig. 2 dargestellten Schaltposition in die jeweils andere Schaltposition gebracht werden. Durch Betätigen des Umschalters 98a werden die Ausgangssignale der Frequenzteiler-Schaltung 84 von dem elektronischen Umschalter 88 ferngehalten. Statt dessen wird dieser auf ein logisch "O" entsprechendes Potential gelegt, so daß er seine in Fig. 2 dargestellte Schaltposition beibehält. Der Ausgang 83 der Gebereinrichtung 80 ist also stets mit der Impulsverzögerungsschaltung 86 verbunden. Da das Hochspannungsrelais 78 über den Steuereingang 79 der Generatoreinrichtung 70 weiterhin im Takt des Ausgangssignals der Frequenzteiler-Schaltung 84 umgeschaltet wird, wird dann abwechselnd jeweils eine Stoßwelle hoher und eine Stoßwelle geringer Intensität erzeugt, die jeweils in der gleichen ortsfesten Fokuszone zusammenlaufen. Mittels des Umschalters 98b wird die Empfangsschaltung 93 derart umgeschaltet, daß sie die mittels der Kunststoffolie 24 aufgenommenen akustischen Echosignale der Stoßwellenquelle geringer Intensität im A-Mode auswertet, so daß auf dem Datensichtgerät 94 ein einem Ultraschall-A-Bild ähnelndes Bild der Fokuszone der Stoßwellen hoher Intensität dargestellt wird.

Wird außerdem der Umschalter 99 ausgehend von seiner in der Fig. 2 dargestellten Schaltposition geöffnet, sind die Ausgangssignale der Frequenzteiler-Schaltung 84 von dem Steuereingang 79 ferngehalten, so daß ausschließlich Stoßwellen hoher Intensität erzeugt werden, anhand von deren akustischen Echosignalen mittels der Kunststoffolie 24 und der Empfangsschaltung 93 ebenfalls Ultraschall-A-Bildern ähnelnde Bilder, die auf dem Datensichtgerät 94 dargestellt werden, erzeugt werden.

Besitzt die Frequenzteiler-Schaltung 84 einen von zwei abweichenden Teilerfaktor, z.B. den Teilerfaktor 8, und nimmt der Umschalter 99 seine in Fig. 2 gezeigte Schaltposition ein, werden abweichend von dem oben Gesagten zunächst sieben Stoßwellen hoher Intensität erzeugt, bevor die Erzeugung der Folge von Stoßwellen geringer Intensität bzw. einer einzelnen Stoßwelle geringer Intensität erfolgt.

Wie aus der Fig. 1 ersichtlich ist, enthält die Ablenkeinrichtung 29 als wesentlichste Bauteile ein Paar von kreisscheibenförmigen Körpern 30, 31, deren Mittelachsen der Mittelachse der Einrichtung entsprechen. Jeder der Körper 30, 31 besitzt eine ebene Begrenzungsfläche 32, 33, die rechtwinklig zur Mittelachse M der Stoßwellenquelle verläuft, und eine ebene Begrenzungsfläche 34, 35, die in bezug auf die Mittelachse M der Stoßwellenquelle 2, deren Richtung der Ausbreitungsrichtung der Stoßwellen vor der akustischen Ablenkeinrichtung 29 entspricht, um einen Winkel geneigt ist, wobei die Begrenzungsflächen 34, 35 jeweils um den gleichen Winkel geneigt sind. Die Körper 30, 31, die übrigens die gleichen Abmessungen aufweisen, sind also von keilförmiger Gestalt. Der Durchmesser der Körper 30, 31 ist so gewählt, daß sie die Querschnittsfläche der Stoßwellen im Bereich der akustischen Ablenkeinrichtung 29 abdecken. Die Körper 30, 31 sind auf einer sich mit der Mittelachse M der Stoßwellenquelle 2 fluchtend in Richtung auf die akustische Linse 14 erstreckenden Achse 36 mit Hilfe einer abgedichteten Wälzlager-Anordnung 37 unabhängig voneinander drehbar gelagert.

Die Körper 30, 31 können mit Hilfe von Zahnriemen 48, 49 in Drehung versetzt werden, die in Verzahnungen 50, 51 gleichen Durchmessers eingreifen, die jeweils am unteren Ende von rohrförmigen, drehfest mit den Körpern 30 bzw. 31 verbundenen Bauteilen 38 bzw. 43 vorgesehen sind. Ein in einer seitlichen Ausbauchung des Gehäuses 1 angeordneter Elektromotor 52 treibt zwei Zahnriemenräder 53, 56 gleichen Durchmessers an, mit denen die Zahnriemen 48 bzw. 49 in Eingriff stehen. Auf der Antriebswelle des Elektromotors 52 ist außer dem Zahnriemenrad 53 ein gewöhnliches Zahnrad 54 angebracht, das mit einem auf einer mit dem Gehäuse 1 verbundenen Achse drehbar gelagerten Zahnrad 55 gleichen Durchmessers kämmt. Mit dem Zahnrad 55 ist das Zahnriemenrad 56 drehfest verbunden. Die Körper 30, 31 werden somit mittels des Elektromotors 52 über die Zahnriemen 48, 49 mit gleicher Winkelgeschwindigkeit in entgegengesetzter Drehrichtung gedreht. Während einer vollständigen Umdehung der Körper 30, 31 ergeben sich dabei zwei Positionen, in denen die Begren zungsflächen 34, 35 parallel zueinander verlaufen. Die Phasenlage zwischen den Körpern 30, 31 kann verändert werden, indem die Winkelposition des Zahnrades 55 relativ zu dem Zahnriemenrad 56 verändert wird. Dies kann wie in Fig. 1 dargestellt geschehen, indem das Zahnrad 55 an seiner dem Zahnriemenrad 56 zugewandten Stirnfläche eine Vielzahl von Bohrungen 58, z.B. in Winkelabständen von 15°, aufweist, und das Zahnriemenrad 56 mit einem Vorsprung 57 mit der der jeweils gewünschten Phasenlage zwischen den Körpern 30, 31 entsprechenden Bohrung 58 in Eingriff gebracht wird. Auf diese Weise ist zugleich eine drehfeste Verbindung zwischen dem Zahnrad 55 und dem Zahnriemenrad 56 hergestellt.

Die keilförmigen Körper 30, 31 bestehen aus Polymethylpentene (TPX) und sind von einem feststehenden Gehäuse 60 aus dem gleichen Werkstoff umgeben. Mit Hilfe eines Dichtringes 61 ist der die Körper 30, 31 enthaltende Innenraum des Gehäuses 60 flüssigkeitsdicht abgeschlossen. Er enthält eine Fluor-Kohlenstoff-Flüssigkeit, z.B. Flutec PP3 oder Fluorinert FC 75. Während die Schallausbreitungsgeschwindigkeit in dem Werkstoff der Körper 30, 31 ca. 2000 m/s beträgt, liegt die Schallausbreitungsgeschwindigkeit in der im Innenraum des Gehäuses 60 befindlichen, die Körper 30, 31 umgebenden Flüssigkeit in der Größenordnung von 600 m/s. Infolge dieser unterschiedlichen Schallausbreitungsgeschwindigkeiten werden Stoßwellen, die die akustische Ablenkeinrichtung 29 durchlaufen, gebrochen. Infolge des Umstandes, daß die Körper 30, 31 in einer Fluor-Kohlenstoff-Flüssigkeit und nicht direkt in dem in dem Gehäuse 1 enthaltenen Wasser angeordnet sind, was an sich auch denkbar wäre, tritt eine stärkere Brechungswirkung auf, da die Schallausbreitungsgeschwindigkeit in der Fluor-Kohlenstoff-Flüssigkeit von der in den Körpern 30, 31 stärker abweicht als die in Wasser, die bei 1500 m/s liegt.

Nehmen die Körper 30, 31 eine Position ein, wie sie in der Fig. 1 dargestellt ist, wird die Fokuszone der Stoßwellen um das größtmögliche Maß verlagert, und zwar derart, daß sie sich in der Position F1 befindet. Werden die Körper 30, 31 ausgehend von der dargestellten Position gegensinnig zueinander verdreht, wandert die Fokuszone der Stoßwellen allmählich zur Mittelachse M der Stoßwellenquelle 2. Haben beide Körper 30, 31 eine viertel Umdrehung ausgeführt, liegen ihre Begrenzungsflächen 34, 35 parallel zueinander und die Fokuszone der Stoßwellen nimmt demgemäß die Position F auf der Mittelachse M der Stoßwellenquelle 2 ein. Während der nächsten viertel Umdrehung der Körper 30, 31 wandert die Fokuszone in die Position F2, wobei die Körper 30, 31 eine Lage einnehmen, die dem Spiegelbild der gezeigten Lage entspricht. Nach einer weiteren viertel Umdrehung der Körper 30, 31 verlaufen deren Begrenzungsflächen 34, 35 wieder parallel zueinander, was bedeutet, daß die Fokuszone wieder auf der Mittelachse M der Stoßwellenquelle 2 zu liegen kommt. Vollenden die Körper 30, 31 eine volle Umdrehung, wandert die Fokuszone in die Ausgangsposition F1. Dies wiederholt sich bei jeder vollen Umdrehung der Körper 30, 31. Es wird somit deutlich, daß es mittels der akustischen Ablenkeinrichtung 29 möglich ist, die Fokuszone der Stoßwellen wie bereits erwähnt zur Erzeugung eines Sektor-Scans abzulenken.

Um die Abgabe von Stoßwellen hoher bzw. geringer Intensität in der erforderlichen Weise mit der Bewegung der Körper 30, 31 und damit der Bewegung der Fokuszone der Stoßwellen zeitlich abstimmen zu können, ist die bereits erwähnte Gebereinrichtung 80 vorgesehen. Diese umfaßt zwei Hallgeneratoren 80a, 80b, von denen einer ortsfest an dem Boden und einer ortsfest an dem Deckel des Gehäuses 60 angebracht ist. Außerdem umfaßt die Gebereinrichtung vier Permanentmagnete 80c, 80d bzw. 80e, 80f, von denen je zwei an einander diametral gegenüberliegenden Stellen an dem Körper 30 bzw. an dem Körper 31 derart angebracht sind, daß sie während der Drehung der Körper 30, 31 an den Hallgeneratoren 80a bzw. 80b vorbeibewegt werden. Pro Umdrehung der Körper 30, 31 liefert der Hallgenerator 80a demgemäß zwei Impulse, die an dem Ausgang 82 der Gebereinrichtung 80 zur Verfügung stehen. Aus der Zeitdifferenz zwischen diesen Impulsen und den von dem Hallgenerator 80b gelieferten Impulsen wird das am Ausgang 83 der Gebereinrichtung 80 zur Verfügung stehende impulsartige Signal gebildet, das jedesmal dann auftritt, wenn die Fokuszone eine bestimmte Position einnimmt, wobei diese Position von der jeweils eingestellten Phasenlage der Körper 30, 31 zueinander abhängt. Es ist daher erforderlich, die Verzögerungszeiten der Impulsverzögerungsschaltungen 86, 87 mittels der Einstellwiderstände 91, 92 an die jeweils eingestellte Phasenlage der Körper 30, 31 anzupassen, um zu erreichen, daß die Stoßwellen hoher Intensität in der jeweils gewünschten ortsfesten Fokuszone zusammenlaufen und der Sektor-Scan der Stoßwellen geringer Intensität den gewünschten Sektor erfaßt.

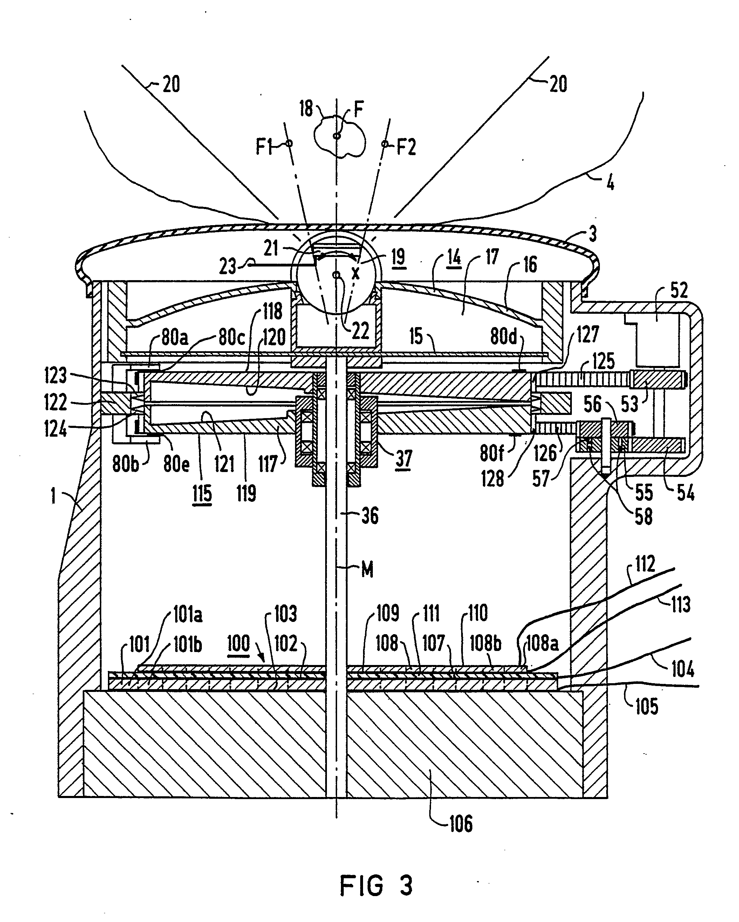

Die in Fig. 3 dargestellte erfindungsgemäße Einrichtung weicht von der zuvor beschriebenen Einrichtung nur hinsichtlich der Ausbildung der akustischen Ablenkeinrichtung und der Stoßwellenquelle ab, weshalb jeweils gleiche Teile die gleichen Bezugszeichen tragen.

Anstelle der elektro-dynamischen Stoßwellenquelle 2 gemäß Fig. 1 ist eine insgesamt mit 100 bezeichnete piezoelektrische Stoßwellenquelle vorgesehen. Diese weist eine erste piezokeramische Schicht 101 auf, die z.B. aus Barium-Titanat bestehen kann. Diese ist an ihren beiden Stirnflächen mit Metallschichten 102, 103, die z.B. aus Blei bestehen, versehen, die zur Kontaktierung der piezoelektrischen Schicht dienen und an die Leitungen 104, 105 angeschlossen sind, die zu einer entsprechend Fig. 2 ausgebildeten Generatoreinrichtung führen. Die piezoelektrische Schicht 101, die demgemäß zur Erzeugung von Stoßwellen hoher bzw. niedriger Intensität dient, ist mit ihrer Metallisierung 103 an einem Dämpfungskörper 106 befestigt. Die von dem Dämpfungskörper 106 abgewandte Stirnfläche der piezoelektrischen Schicht 101 bzw. die mit dieser verbundene Metallschicht 102 bildet die Abstrahlfläche 107 der Stoßwellenquelle 100. Auf die Abstrahlfläche 107 ist eine zweite piezokeramische Schicht 108, die ebenfalls aus Barium-Titanat bestehen kann, aufgebracht. Auch diese ist zur elektrischen Kontaktierung an ihren beiden Stirnflächen mit z.B. aus Blei bestehenden Metallschichten 109, 110 versehen, wobei zwischen den Metallschichten 102 und 109 eine Isolierschicht 111 vorgesehen ist. Die zweite piezokeramische Schicht 108 wirkt als Drucksensor zum Empfang der akustischen Echosignale der von der Abstrahlfläche 107 ausgehenden Stoßwellen und ist demgemäß über an den Metallschichten 109, 110 angebrachte Leitungen 112, 113 mit einer der Empfangsschaltung 93 gemäß Fig. 2 entsprechenden Empfangsschaltung verbunden. Die zweite piezokeramische Schicht 108 weist eine wesentlich geringere Dicke als die erste piezokeramische Schicht 101 auf. Sie besitzt damit eine deutlich höhere Resonanzfrequenz als die erste piezokeramische Schicht 101. Sowohl die erste piezokeramische Schicht 101 als auch die zweite piezokeramische Schicht 108 können aus einzelnen piezokeramischen Elementen 101a, 101b usw. bzw. 108a, 108b usw. mosaikartig zusammengesetzt sein, was in Fig. 3 schematisch angedeutet ist.

Die im Falle der Fig. 3 vorgesehene akustische Ablenkeinrichtung 115 weist wieder zwei keilförmige Körper 116, 117 auf, die aus Polymethylpentene (TPX) gebildet sind. Im Gegensatz zu der zuvor beschriebenen akustischen Ablenkeinrichtung 29 sind die Körper 116, 117 jedoch nicht in einem Gehäuse aufgenommen. Vielmehr grenzen deren rechtwinklig zur Mittelachse M der Stoßwellenquelle 100 verlaufende Begrenzungsflächen 118, 119 unmittelbar an das in dem Gehäuse 1 befindliche Wasser. Lediglich zwischen den einander zugewandten in bezug auf die Mittelachse M der Stoßwellenquelle 100 jeweils um den gleichen Winkel geneigten Begrenzungsflächen 120, 121 ist eine Fluor-Kohlenstoff-Flüssigkeit vorhanden. Um zu vermeiden, daß sich diese mit dem in dem Gehäuse 1 befindlichen Wasser vermischt, ist ein mit dem Gehäuse 1 fest verbundenes ringförmiges Bauteil 122 vorgesehen, das die Körper 116, 117 an deren Umfang umgibt und mit dessen Bohrung an den Umfangsrändern der Körper 116, 117 angebrachte Dichtringe 123, 124 in Eingriff stehen.

Der Antrieb der Körper 116, 117 erfolgt wieder mit Hilfe von Zahnriemen 125, 126, die im Falle der Fig. 3 mit Verzahnungen 127, 128 zusammenwirken, die am Umfang der Körper 116, 117 vorgesehen sind.

Auch im Falle der Fig. 3 sind Hallgeneratoren 80a, 80b vorgesehen, die an dem ringförmigen Bauteil 122 angebracht sind und mit an den Körpern 116 bzw. 117 angebrachten Permanentmagneten 80c, 80d bzw. 80e, 80f zusammenwirken.

Die akustische Ablenkeinrichtung 115 weist einen Abstand von der Abstrahlfläche 107 der Stoßwellenquelle 100 auf, der etwa der Fokuslänge der akustischen Sammellinse 14 entspricht. Durch diese Maßnahme ist sichergestellt, daß zwischen der Abstrahlfläche 107 der Stoßwellenquelle 100 und der akustischen Ablenkeinrichtung 115 auftretende Mehrfachechos an die Unterkante des mittels der Stoßwellen geringer Intensität erzeugten Bildes verlagert sind. Eine entsprechende Anordnung ist übrigens auch im Falle der erfindungsgemäßen Einrichtung nach Fig. 1 gewählt.

Im Falle der Einrichtung nach der Fig. 3 kann anstelle der zweiten piezokeramischen Schicht ähnlich wie im Falle der Fig. 1 eine piezoelektrisch aktivierte Kunststoffolie als Drucksensor vorgesehen sein. Diese sollte dann jedoch in einem Abstand von z.B. zwei bis drei Zentimetern von der Abstrahlfläche 107 der Stoßwellenquelle 100 entfernt angeordnet sein, um nachteilige Auswirkungen von in der Stoßwellenquelle 100 entstehenden Mehrfach-Echos zu vermeiden.

In den Fig. 1 und 2 sind jeweils Stoßwellenquellen 2 bzw. 100 mit ebener Abstrahlfläche 13 bzw. 107 dargestellt. An deren Stelle können auch Stoßwellen mit Abstrahlflächen treten, von denen bereits fokussierte Stoßwellen ausgehen. Es erübrigt sich dann, eine akustische Sammellinse 14 vorzusehen.

## Ansprüche

1. Einrichtung zum berührungslosen Zertrümmern von Konkrementen (18) im Körper (4) eines Lebewesens mit einer Stoßwellenquelle (2; 100) zur Erzeugung von Stoßwellen in einem akustischen Ausbreitungsmedium, welche in einer Fokuszone (F) zusammenlaufen, wobei die Stoßwellenquelle (2; 100) wahlweise zur Erzeugung von Stoßwellen hoher Intensität zur Zertrümmerung eines Konkrementes (18) und zur Erzeugung von Stoßwellen geringer Intensität für bildgebende Zwecke antreibbar ist, mit Mitteln (3) zur akustischen Koppelung der Stoßwellenquelle (2; 100) mit dem Körper (4) des Lebewesens, und mit einem Drucksensor (24; 108), der die akustischen Echosignale der Stoßwellen empfängt und der zur Auswertung der Echosignale mit einer Empfangsschaltung (93) verbunden ist, **dadurch gekennzeichnet,** daß der Drucksensor (24; 108) in dem Ausbreitungsmedium zwischen der Fokuszone (F) und der Stoßwellenquelle (2; 100) angeordnet ist und eine Sensorfläche aufweist, die die Querschnittsfläche der Stoßwellen im Bereich des Drucksensors (24; 108) abdeckt, daß zwischen dem Drucksensor (24; 108) und der Fokuszone (F) eine akustische Ablenkeinrichtung (29; 115) in dem Ausbreitungsmedium angeordnet ist, mittels derer die Stoßwellen geringer Intensität zur Erzielung eines die Fokuszone (F) der Stoßwellen hoher Intensität erfassenden Sektor-Scans ablenkbar sind, und daß die Empfangsschaltung (93) zur Erzeugung von B-Bildern betreibbar ist.

2. Einrichtung nach Anspruch 1, **dadurch gekennzeichnet,** daß die Stoßwellenquelle (2; 100) antreibbar ist, daß sie zwischen jeweils zwei Stoßwellen hoher Intensität eine Vielzahl von Stoßwellen geringer Intensität bei hoher Folgefrequenz erzeugt.

3. Einrichtung nach Anspruch 1 oder 2, **dadurch gekennzeichnet,** daß die akustische Ablenkeinrichtung (29; 115) ein Paar von keilförmigen Körpern (30, 31; 116, 117) aufweist, die aus einem Werkstoff mit von der eines sie umgebenden akustischen Ausbreitungsmediums abweichender Schallausbreitungsgeschwindigkeit gebildet sind und in Ausbreitungsrichtung der Stoßwellen aufeinanderfolgend um Drehachsen (M) relativ zueinander drehbar angeordnet sind, wobei jeder keilförmige Körper (30, 31; 116, 117) eine in bezug auf die Ausbreitungsrichtung der Stoßwellen um jeweils den gleichen Winkel geneigte und jeweils die Querschnittsfläche der Stoßwellen im Bereich der akustischen Ablenkeinrichtung (29; 115) abdeckende Begrenzungsfläche (34, 35; 120, 121) aufweist, und daß Mittel (48, 49, 50, 51, 52, 53, 54, 55, 56; 125, 126, 127, 128) zum Drehen der keilförmigen Körper (30, 31; 116, 117) um die jeweilige Drehachse (M) vorgesehen sind, mittels derer die keilförmigen Körper (30, 31; 116, 117) in entgegengesetzter Richtung mit gleicher Winkelgeschwindigkeit antreibbar sind, so daß während einer Umdrehung der keilförmigen Körper (30, 31; 116, 117) die Begrenzungsflächen (34, 35; 120, 121) in zwei Winkelpositionen parallel zueinander liegen.

4. Einrichtung nach den Ansprüchen 2 und 3, **dadurch gekennzeichnet,** daß eine Gebereinrichtung (80, 80a, 80b, 80c, 80d, 80e, 80f) vorgesehen ist, die bei einer definierten Lage der keilförmigen Körper (30, 31; 116, 117) zueinander ein Signal abgibt, daß zwischen die Gebereinrichtung (80, 80a, 80b, 80c, 80d, 80e, 80f) und eine die Stoßwellenquelle (2; 100) antreibende Generatoreinrichtung (70) eine Verzögerungsschaltung (87) geschaltet ist, die das Signal der Gebereinrichtung (80, 80a, 80b, 80c, 80d, 80e, 80f) um eine Verzögerungszeit verzögert, und daß das verzögerte Signal die Generatoreinrichtung 70) veranlaßt, die Stoßwellenquelle (2; 100) zur Abgabe der Vielzahl von Stoßwellen geringer Intensität anzutreiben, wobei die Verzögerungszeit derart bemessen ist, daß ein Sektor-Scan erzielt wird, der die Fokuszone (F) der Stoßwellen hoher Intensität erfaßt.

5. Einrichtung nach einem der Ansprüche 2 bis 4, **dadurch gekennzeichnet,** daß die Phasenlage der keilförmigen Körper (30, 31; 120, 121) relativ zueinander verstellbar ist.

6. Einrichtung nach einem der Ansprüche 1 bis 5, **dadurch gekennzeichnet,** daß die Stoßwellenquelle (2; 100) im wesentlichen ebene Stoßwellen erzeugt, daß zur Fokussierung der Stoßwellen eine akustische Sammellinse (14) vorgesehen ist, und daß die Ablenkeinrichtung (29; 115) zwischen dem Drucksensor (24; 108) und der akustischen Sammellinse (14) angeordnet ist.

7. Einrichtung nach Anspruch 6, **dadurch gekennzeichnet,** daß die akustische Ablenkeinrichtung (29; 115) in einem Abstand von der Stoßwellenquelle (2; 100) angeordnet ist, der wenigstens der Brennweite der akustischen Sammellinse (14) entspricht.

8. Einrichtung nach einem der Ansprüche 1 bis 7, **dadurch gekennzeichnet,** daß als Drucksensor (24) eine piezoelektrisch aktivierte Kunststoffolie und als Stoßwellenquelle (2) eine stoßartig antreibbare Membran (5) vorgesehen ist, wobei die Kunststoffolie auf der der Fokuszone (F) zugewandten Seite der Membran (5) aufgebracht ist.

9. Einrichtung nach einem der Ansprüche 1 bis 7, **dadurch gekennzeichnet,** daß als Stoßwellenquelle (100) eine piezoelektrische Stoßwellenquelle vorgesehen ist, die eine erste piezoelektrische Schicht (101) zur Erzeugung der Stoßwellen und eine auf der ersten piezoelektrischen Schicht (101) als Drucksensor (108) aufgebrachte zweite piezoelektrische Schicht aufweist, die im Sinne der Ausbreitungsrichtung der Stoßwellen nach der ersten piezoelektrischen Schicht (101) angeordnet ist und eine höhere Resonanzfrequenz aufweist als die erste piezoelektrische Schicht (101).

10. Einrichtung nach einem der Ansprüche 1 bis 9, **dadurch gekennzeichnet,** daß die mit dem Drucksensor (24; 108) empfangenen Echosignale eine Stoßwelle hoher oder geringer Intensität mittels der Empfangsschaltung (93) im A-Mode auswertbar sind.

**FIG 1**

FIG 2

FIG 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.3) |
|---|---|---|---|
| A | EP-A-0 254 104 (SIEMENS)<br>* Spalte 4, Zeilen 8-27; Spalte 9, Zeile 6 - Spalte 10, Zeile 6; Figur 8 *<br>--- | 1,2,8,9 | A 61 B 17/22<br>G 10 K 11/30 |
| A,D | DE-B-2 722 252 (DORNIER)<br>* Spalte 2, Zeilen 44-60; Spalte 5, Zeile 3 -Spalte 6, Zeile 15; Ansprüche 1,4,6; Figuren 1-5 *<br>--- | 1,8-10 | |
| A | FR-A-2 591 467 (WOLF)<br>* Seite 1, Zeile 1 - Seite 2, Zeile 3; Seite 4, Zeile 13 - Seite 5, Zeile 27; Ansprüche 1,2; Figuren 11-13 *<br>--- | 1,2 | |
| A | US-A-4 131 021 (MEZRICH et al.)<br>* Spalte 9, Zeile 23 - Spalte 10, Zeile 39; Ansprüche 1,2,4; Figur 4 *<br>--- | 3-7 | |
| A,D | US-A-3 913 061 (GREEN)<br>* Spalte 5, Zeile 16 - Spalte 6, Zeile 57; Spalte 7, Zeilen 18-62; Figur 1 *<br>----- | 3-7 | RECHERCHIERTE SACHGEBIETE (Int. Cl.3)<br><br>A 61 B 17/00<br>G 10 K 11/00 |

Der vorliegende Recherchenbericht wurde für alle Patentansprüche erstellt

| Recherchenort | Abschlußdatum der Recherche | Prüfer |
|---|---|---|
| BERLIN | 30-03-1989 | MONNE E.M.B. |